(19) Europäisches Patentamt
European Patent Office
Office européen des brevets

(11) **EP 3 712 786 A1**

(12) **EUROPÄISCHE PATENTANMELDUNG**

(43) Veröffentlichungstag:
**23.09.2020 Patentblatt 2020/39**

(51) Int Cl.:
***G06F 17/18*** *(2006.01)*

(21) Anmeldenummer: **20164529.8**

(22) Anmeldetag: **20.03.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **21.03.2019 DE 102019203895**

(71) Anmelder: **Robert Bosch GmbH**
**70442 Stuttgart (DE)**

(72) Erfinder:
• **Schreivogel, Martin**
**70376 Stuttgart (DE)**
• **Ulrich, Markus**
**70374 Stuttgart (DE)**

(54) **VERFAHREN ZUM AUSWERTEN MINDESTENS EINES SIGNALS**

(57) Verfahren zum Auswerten mindestens eines Signals, das eine Zeitreihe von Messwerten repräsentiert, wobei die Messwerte Immissionen betreffen, wobei das mindestens eine Signal mittels einer trendbereinigenden Fluktuationsanalyse ausgewertet wird, so dass ein trendbereinigtes Signal erhalten wird, das wiederum ausgewertet wird, um eine zeitliche Veränderung der Fluktuation der Messwerte zu erkennen und diese zeitliche Veränderung auszuwerten.

**Fig. 6**

**Beschreibung**

Die Erfindung betrifft ein Verfahren zum Auswerten mindestens eines Signals und eine Anordnung zum Durchführen des Verfahrens.

Stand der Technik

Es ist bekannt, Luftverschmutzungen, die durch Emissionen, wie bspw. Stickoxid, Kohlenmonoxid, Schwefeldioxid, Ozon, Partikelemissionen usw., bedingt sind, mittels aufwändiger und stationärer Messpunkte zu erfassen. Die Emissionen werden beim Betrieb von Fahrzeugen, allgemein durch Verkehr, von Industrieanlagen und von Haushalten ausgestoßen. Daher finden diese Messungen insbesondere in Ballungsgebieten statt. Bei der Messung wird der Ist-Zustand der an einer Messstation gemessenen Immissionen erfasst und eine Prognose der zu erwartenden Immissionen oftmals auf Basis des Ist-Zustands bzw. ggf. vorhandener Trends sowie anhand von Wetter-Prognosen getroffen. Diese Prognosen bilden die messtechnische Grundlage von strategischen Eingriffen, wie z. B. einem Feinstaubalarm.

Unter Immission ist die Einwirkung von Verunreinigungen der Luft, des Bodens und des Wassers auf lebende Organismen oder Gegenstände zu verstehen. Immissionen hängen von Emissionen ab, allerdings weder eindeutig noch unmittelbar, da diese auch von anderen Einflussgrößen, wie bspw. Wetterbedingungen, abhängig sind.

Zu berücksichtigen ist, dass die Immissionsmesswerte räumlich sowie zeitlich relativ grob abgetastet und in der Regel stündliche oder tägliche Mittelwerte berechnet werden, die dann für weitere Prognosen und für Überwachungszwecke genutzt werden. Vermehrt wird hierbei jedoch auch auf dezentrale, verteilte Lösungen gesetzt, um ein größeres Gebiet feinmaschiger zu messen. Die Auswertungen verlaufen hier entsprechend.

Offenbarung der Erfindung

Vor diesem Hintergrund werden ein Verfahren nach Anspruch 1 und eine Anordnung gemäß Anspruch 9 vorgestellt. Ausführungsformen ergeben sich aus den abhängigen Ansprüchen und aus der Beschreibung.

Das vorgestellte Verfahren dient zum Auswerten mindestens eines Signals, das eine Zeitreihe von Messwerten, die Immissionen betreffen, umfasst. Das mindestens eine Signal wird mittels einer trendbereinigenden Fluktuationsanalyse, bspw. einer DFA (DFA, engl. Detrended Fluctuation Analysis), analysiert. Dies bedeutet, dass Daten bzw. Messwerte bereitgestellt werden, die kontinuierlich oder in zeitlichen Abständen gemessen wurden. Diese Daten bzw. Messwerte liegen in der Reihenfolge, in der sie gemessen wurden, vor. Eine Zeitreihe mit den in zeitlicher Reihenfolge vorliegenden Messwerten bzw. Daten wird durch das Signal, das bereitgestellt und hierin auch als Rohsignal bezeichnet wird, repräsentiert. Folglich wurde das Signal in Form einer Zeitreihe, die Messwerte in zeitlicher Reihenfolge umfasst, aufgezeichnet.

Durch die Analyse von Zeitreihen erhofft man sich Aufschlüsse über die zu Grunde liegenden Mechanismen entsprechend betrachteter Systeme. So werden Zeitreihen in Bereichen wie Meteorologie, Hydrologie, Geologie, Medizin, Biologie aber auch Finanzmathematik analysiert, um bspw. Klimaprognosen zu überprüfen, Vulkanaktivitäten frühzeitig in seismischen Daten zu erkennen, Schlafphasen über Änderungen in der Herzratenvariabilität zu identifizieren oder auf den Gesundheitszustand von Patienten zu schließen.

Es ist bekannt, dass in komplexen, stark gekoppelten Systemen Fluktuationen oftmals nicht unabhängig voneinander, sondern langzeitkorreliert sind. Man spricht in diesem Fall der Langzeitkorrelation auch oft von Persistenz, also einer Art Gedächtnis, die den Zustand eines Systems in vielen Fällen über einen überraschend langen Zeitraum aufrechterhält. Ein anschauliches Beispiel aus der Meteorologie liefern Hochdruckgebiete, in denen sich das Wetter oft über Tage stabil hält. Zeitreihen sind in der Regel trendbehaftet, z. B. monotone Trends bei driftenden Systemen oder periodische Trends, wie bspw. Tag/Nacht oder Jahreszeiten.

Konventionelle Methoden zur Untersuchung von Korrelationen in Zeitreihen, wie bspw. die Berechnung der Autokorrelationsfunktion oder die Ermittlung der Leistungsdichte durch eine Fourier-Transformation, führen in diesen instationären Fällen zu Artefakten, die schnell als Korrelationen missinterpretiert werden können. Daher muss bei instationären Zeitreihen zunächst zwischen Trend und Fluktuationen unterschieden und der Trend aus der Zeitreihe entfernt werden. Hierbei hat sich im Laufe der letzten beiden Jahrzehnte die sog. trendbereinigte Fluktuationsanalyse (DFA: Detrended Fluctuation Analysis) etabliert, die Fluktuationen als Bewegungen im Sinne eines Random Walk auffasst, dabei Trends bereinigt und das Skalenverhalten der Fluktuation der trendbereinigten Zeitreihe ermittelt. Es sind aber auch andere geeignete Methoden für eine Fluktuationsanalyse denkbar, wie z. B. eine Wavelet-Analyse oder die ARMA-Methode (ARMA: Autoregressive Moving Average) sowie deren Erweiterungen, wie z. B. ARIMA (Autoregressive Integrated Moving Average) oder aber Methoden des maschinellen Lernens, z. B. neuronale Netze.

Um das Signal bzw. die Zeitreihe von Trends zu befreien, wird in Ausgestaltung eine DFA durchgeführt, so dass ein trendbereinigtes Signal erhalten wird, das dann weiter analysiert wird, um eine zeitliche Veränderung der Fluktuation der Daten zu erkennen und auszuwerten. Dabei ist insbesondere vorgesehen, die zeitliche Veränderung der Fluktuation zu nutzen, um auf zukünftige Ereignisse, die das beobachtete System betreffen, schließen zu können.

Unter Fluktuation ist eine kurzzeitige oder andauernde Veränderung, d. h. eine Schwankung bzw. ein Wechsel, von Gegebenheiten und Zuständen, d. h. in diesem Fall in dem Signal bzw. den Signalwerten, zu verstehen. Ein Trend ist eine systematische Zunahme oder systematische Abnahme in einer Zeitreihe. Diese Zeitreihe kann zudem Fluktuation oder periodische Schwingungen aufweisen. Ein Trend bei der Immissionsmessung kann bspw. durch die Tageszeit gegeben sein. So sind Emissionen und damit Immissionen von der Tageszeit abhängig. Die Auswirkungen solcher Trends sollen nunmehr eliminiert werden, um die Fluktuation zu erfassen bzw. erkennen zu können.

In Folge des wachsenden Bewusstseins um die schädliche Wirkung von Luftbelastungen, insbesondere in Ballungsgebieten und Großstädten, wurde die DFA auch genutzt, um Langzeitkorrelationen in Luftverschmutzungen durch Partikelemissionen zu untersuchen. Die Anwendung auf weitere Schadstoffe, Untersuchungen über längere Zeiträume sowie der Versuch von Prognosen im Sinne eines Frühwarnsystems sind hingegen bislang unbekannt.

Die Prognosen von insbesondere vom Straßenverkehr in Ballungsräumen hervorgerufenen Emissionen werden in vielen Fällen nur auf Basis von Wetterlagen getroffen und bilden nur bedingt die Realität ab. Der zeitliche Verlauf von gemessenen Immissionen aus der Vergangenheit sowie insbesondere deren zeitliche Schwankungen werden regelmäßig nicht berücksichtigt. So können Maßnahmen u. U. unverhältnismäßig lange aufrechterhalten oder aber auch zu früh aufgehoben werden. Zudem ist es möglich, dass auf Basis ungenauer Prognosen Maßnahmen zu einem falschen Zeitpunkt ergriffen werden.

Durch das beschriebene Verfahren können Immissionen zeitlich hoch abgetastet gemessen und Prognosen auf Basis der Änderung von Langzeitkorrelationen im Rohsignal getroffen werden, um Maßnahmen zur Gegensteuerung kurzfristiger sowie räumlich begrenzt einzuleiten bzw. anzupassen, um Immissionen zu reduzieren.

Mit dem hier vorgestellten Vorgehen werden Immissionen zeitlich hoch aufgelöst gemessen, wodurch auch Fluktuationen auf kurzen Zeitskalen erfasst werden. Dies ist eine notwendige Voraussetzung, um Langzeitkorrelationen im Immissionsverhalten einer Atmosphäre zu ermitteln. Die mathematisch ermittelten Langzeitkorrelationen geben nicht nur Aufschluss über die Persistenz einer Luftbelastung, sondern erlauben mit dem vorgestellten mathematischen Verfahren der Auswertung von Korrelationen in kürzeren Zeitfenstern und Bewertung der zeitlichen Änderungen in den Korrelationen auf kürzeren Zeitskalen auch eine Prognose der zu erwartenden Emissionen.

Der einfache Aufbau der hierin vorgestellten Vorrichtung zur Messung von Immissionen ermöglicht, im Gegensatz zu deutlich aufwändigeren und teureren Messstationen, eine räumlich höher aufgelöste Messung über ein Netzwerk von räumlich verteilten Messknoten. Durch den Vergleich der Langzeitkorrelationen an räumlich unterschiedlichen Stellen sowie der Prognosen an unterschiedlichen Stellen können Maßnahmen kurzfristig lokal beschränkt sowie langfristig räumliche Besonderheiten detektiert werden. Die mit dem vorgestellten Verfahren generierten Informationen können zur Planung aber auch zur Bewertung der Wirksamkeit von kurz bis mittelfristigen Maßnahmen, wie bspw. Geschwindigkeitsbegrenzungen, Anpassungen von Ampelphasen oder lokale Durchfahrtsbeschränkungen, bis hin zu langfristigen Maßnahmen, wie bspw. die bauliche Veränderungen im Stadtbild, zur Reduktion von Emissionen herangezogen werden.

Prinzipiell können die Daten darüber hinaus genutzt werden, um z. B. mit Methoden des maschinellen Lernens die Wirkungen von Maßnahmen auf die Persistenz von Luftverschmutzungen zu bewerten und daraus zielführende Strategien abzuleiten. Dies kann in Verbindung von DFA und maschinellem Lernen, wie bspw. Merkmalsextraktion, überwachtes Lernen und genetische Algorithmen, im Sinne eines selbstlernenden regelnden Systems etabliert werden. Dabei kann die ermittelte Langzeitpersistenz im Gegensatz zu der reinen Bewertung von Tagesmittelwerten zu einer schnelleren Regelung eingesetzt werden.

Die vorgestellte Anordnung dient zum Auswerten mindestens eines Signals und somit zur Durchführung des beschriebenen Verfahrens. Die Anordnung dient insbesondere zum Messen und Überwachen von Immissionen.

Weitere Vorteile und Ausgestaltungen der Erfindung ergeben sich aus der Beschreibung und den beiliegenden Zeichnungen.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Kurze Beschreibung der Zeichnungen

Figur 1 zeigt in schematischer Darstellung eine Anordnung zum Erfassen von Messwerten.

Figur 2 zeigt in vier Graphen das Prinzip der DFA.

Figur 3 zeigt in vier Graphen exemplarische Zeitreihen sowie aus den Zeitreihen ermittelten Größen, u. a. aus der DFA.

Figur 4 zeigt die Fluktuationsfunktion für DFA.

Figur 5 zeigt in einem Graphen die Korrelation zwischen der Änderung der Emissions-Tagesmittelwerte und der Änderung der Fluktuationsexponenten am jeweiligen Vortag.

Figur 6 zeigt eine Regelschleife.

Figur 7 zeigt eine Prinzipskizze lokal ermittelter und geregelter Regionen.

Ausführungsformen der Erfindung

Die Erfindung ist anhand von Ausführungsformen in den Zeichnungen schematisch dargestellt und wird nachfolgend unter Bezugnahme auf die Zeichnungen ausführlich beschrieben.

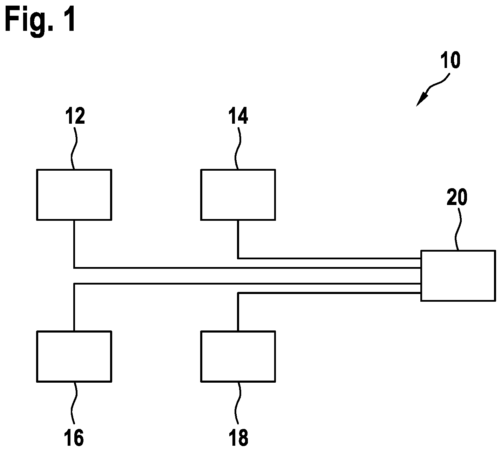

Figur 1 zeigt in schematischer Darstellung eine Messanordnung 10 zum Messen mehrerer Signale, die jeweils Messwerte, die in einer Zeitreihe vorliegen und sich auf Immissionswerte beziehen, umfassen. Die Darstellung zeigt eine erste Messstation 12, eine zweite Messstation 14, eine dritte Messstation 16 und eine vierte Messstation 18, die räumlich voneinander getrennten Messwerte, die Immissionen betreffen, erfassen. Jede Messstation 12, 14, 16, 18 stellt dann ein Signal bereit, das eine Information zu diesen Messwerten trägt. Die Signale der Messstationen werden an eine Anordnung 20 weitergegeben, die die Auswertung des Messsignals gemäß einer Ausführungsform des hierin beschriebenen Verfahrens vornimmt. Diese Anordnung 20 kann auch in einer der Messstationen 12, 14, 16, 18 oder verteilt über die Messstationen 12, 14, 16, 18 vorgesehen sein. Die Anordnung 20 kann als Hardware und/oder Software implementiert sein.

Die Signale werden in Ausführung zeitlich kontinuierlich, äquidistant und möglichst hoch abgetastet und aufgezeichnet. Dabei können durch Einsatz mehrerer Messstationen bzw. Messknoten die Signale auch räumlich an verschiedenen Stellen eines Ballungsgebiets gemessen werden. Die Messstationen zur Messung der Immissionen bestehen dabei aus jeweiligen Sensoren, Betriebselektroniken, einem Datenübertragungsmodul, drahtlos oder kabelgebunden, einer Stromversorgung, typischerweise lokal, bspw. Akku, Solarzelle, und einem Gehäuse mit geeigneter Luftprobennahme.

Die so entstehende Zeitreihe kann entweder lokal in einem internen Datenspeicher zwischengespeichert oder aber über eine integrierte Mobilfunkverbindung an einen Cloud-Server übertragen werden. Hierbei wird eine notwendige Abtastrate, wie bspw. 1 Wert pro Minute oder mehr, durch eine entsprechende Messschaltung sowie Speicher und/oder Datenübertragung sichergestellt. Werden die Signale z. B. alle 10 Sekunden protokolliert, so besteht eine Zeitreihe in der Länge eines Tages aus 8640 einzelnen äquidistanten Datenpunkten. Die Rohdaten der aufgezeichneten Zeitreihe können nun entweder lokal durch eine entsprechend integrierte Rechnerplatine oder im Falle der Datenübertragung an einen Cloud-Server gesendet und extern analysiert werden. Im Falle der lokalen Analyse der Zeitreihe muss nur das Ergebnis der Zeitreihe, neben ggf. gemittelten Werten aus den Rohdaten zum Zweck der Überwachung zusätzlich entweder die Fluktuationsfunktion oder aber nur der Exponent, an einen Cloud-Server übertragen werden. Die Aufteilung der übertragenen bzw. berechneten Daten kann somit zweckmäßig an die örtlichen Möglichkeiten angepasst werden.

Einzelne Zeitreihen eines Tages oder ggf. kürzere oder längere Zeiträume, wobei das Beobachterfenster ggf. auch entlang der Zeitachse verschoben werden kann, oder auch Zeitreihen mit mehreren Tagen Länge können nun hinsichtlich ihres Fluktuationsverhaltens z. B. mit Hilfe trendbereinigender Fluktuationsanalyseverfahren, wie bspw. der DFA, auf Langzeitkorrelationen untersucht werden.

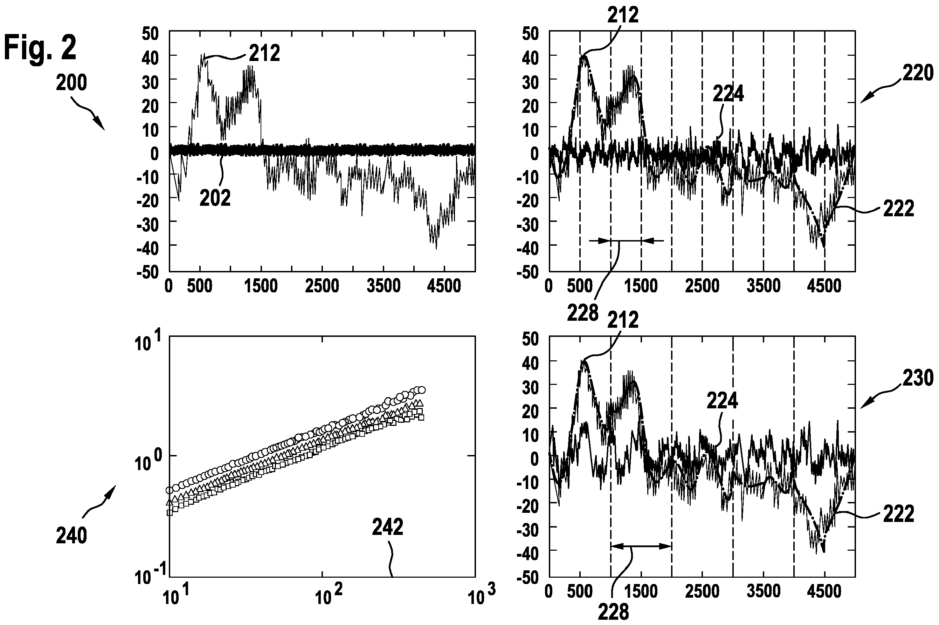

Im Folgenden soll zunächst die DFA als eine mögliche Methode zur Analyse der in der Zeitreihe befindlichen Langzeitkorrelation auch in Verbindung mit Figur 2 beschrieben werden.

Bei der DFA wird das sog. Skalenverhalten der Fluktuation einer Zeitreihe ermittelt. Dabei wird zwischen dem Trend in der Zeitreihe, bspw. jahreszeitliche Trends oder Tag/Nacht-Unterschiede, unterschieden und die Zeitreihe trendbereinigt, um ausschließlich das Verhalten der Fluktuation zu bewerten.

Es wird die Zeitreihe $x_i = \{x_1, \ldots , x_N\}$ aus N äquidistanten Beobachtungswerten betrachtet, z. B. $N = 8640$ Datenpunkte bei einer Zeitreihe von 1 Tag, wobei alle 10 Sekunden ein Wert ermittelt wurde, und der Mittelwert gebildet.

$$\langle x \rangle = \frac{1}{N} \sum_{i=1}^{N} x_i$$

Hieraus wird die neue Zeitreihe $\bar{x}_i \equiv x_i - \langle x \rangle$ durch Abzug des Mittelwerts von der ursprünglichen Zeitreihe erzeugt, wobei dieser Schritt optional ist und nur der Zentrierung dient, und diese kumuliert

$$Y_j = \sum_{i=1}^{j} \bar{x}_i$$

und die kumulierte Reihe in $N_s = N/s$ Segmente der Breite s zerteilt. In jedem Segment $v$ ($v = \{1, \ldots, N_s\}$) wird die Zeitreihe durch ein Polynom $P_v$ $n$-ten Grades approximiert und dieses vom Rohsignal abgezogen $Y_s (i) = Y_i - P_v (i)$. Hierdurch wird das Signal schließlich trendbereinigt. Von dem so erhaltenen Residuum $Y_s$ wird nun die Varianz in jedem Segment v wie folgt berechnet

$$F_s^2(v) = \langle Y_s^2(i) \rangle = \frac{1}{s} \sum_{i=1}^{s} Y_s^2 [(v-1)s + i]$$

und über die Anzahl der Segmente für eine gegebene Segmentbreite gemittelt. Dies wird für verschiedene Segmentbreiten s wiederholt. Trägt man dann die sog. Fluktuationsfunktion

$$F^{(n)}(s) = \sqrt{\frac{1}{N_s} \sum_{v=1}^{N_s} F_s^2(v)}$$

auf, wobei sich die Ordnung n auf die Ordnung des verwendeten Polynoms zur Trendbereinigung bezieht, so zeigt diese im asymptotischen Fall ein Potenzgesetz der Form $F^{(n)}(s) \propto s^{\alpha(n)}$, wobei $\alpha(n)$ der sogenannte Fluktuationsexponent oder auch Hurst-Exponent für eine einfache Fluktuationsanalyse, d. h. ohne vorherige Trendbereinigung und somit n = 0, mit zunehmender Ordnung n gegen einen bestimmten Wert $\alpha$ konvergiert. Es wird hierzu auf Figur 2 verwiesen.

Figur 2 verdeutlicht in vier Graphen das Prinzip einer DFA, wobei dies hier exemplarisch für den Fall eines mittelwertstabilen unkorrelierten Rauschens vorgenommen wird. In einem ersten Graphen 200 ist der Verlauf eines mit Fluktuation behafteten Signals y(t) 202 gezeigt, bspw. eine Messgröße über die Zeit, welche ebenfalls trendbehaftet sein kann. Dieses Signal 202, das auch als Rohsignal 202 bezeichnet wird, wird kumuliert, d. h. sukzessive aufaddiert, so dass sich eine kumulierte Reihe 212 ergibt, deren Verlauf in den Graphen 200, 220 und 230 dargestellt ist. Anschließend wird die kumulierte Reihe 212, was in einem zweiten Graphen 220 dargestellt ist, sukzessive in Fenster der Breite s 228 segmentiert und in jedem Fenster wird die kumulierte Reihe 212 durch ein Polynom n-ten Grades angepasst. Diese Polynome sind durch eine Kurve 222 dargestellt. Es wird dann ein Residuum 224 zwischen kumulierter Reihe 212 und Polynom 222 ermittelt, und zwar durch Subtraktion kumulierte Reihe 212 minus Polynom 222 und die Fluktuation des Residuums 224 in jedem Fenster der Breite s 228 berechnet. Dies wird für verschiedene Fensterbreiten wiederholt, wie in einem dritten Graphen 230 zu sehen ist. Von diesem Residuum 224 wird in jedem Segment die Varianz berechnet und über die verschiedenen Segmente gemittelt. Das Ergebnis wird als Funktion der Fensterbreiten aufgetragen. Es wird hierzu auf einen vierten Graphen 240 verwiesen, an dessen Abszisse 242 die Fensterbreite s aufgetragen ist. Die sogenannte Fluktuationsfunktion $F^{(n)}(s)$, die das Skalenverhalten der Fluktuation der Fensterbreite s darstellt, zeigt dann im Langzeitverhalten ein Potenzgesetz $F^{(n)}(s) \propto s^{\alpha}$ und der Exponent des Langzeitverhaltens $\alpha$ kann zur Identifikation von Langzeitkorrelationen und damit ggf. von zugrundeliegenden Prozessen, wie bspw. Diffusionsund/oder Reaktionsprozessen, die z. B. spezifisch für einen vorliegenden zu detektierenden Stoff sein können, herangezogen werden. Bei dem Beispiel in Figur 2 ergibt sich für $\alpha$ ein Wert von ½. Somit ist die Steigung des asymptotischen Astes der Fluktuationsfunktion der Fluktuationsexponent. Ist $\alpha$ = ½, ist die Reihe unkorreliert, d.

h. weißes Rauschen. Ist $\alpha$ > ½, so ist die Reihe positiv korreliert. Ist $\alpha$ < ½, ist die Reihe negativ korreliert, d. h. antikorreliert.

Lineare Trends werden z. B. durch eine Trendbereinigung mit Polynomen zweiter Ordnung, quadratische Trends hingegen mit Polynomen dritter Ordnung eliminiert. Höhere Trends erfordern somit u. U. höhere Ordnungen in den Polynomen zur Trendbereinigung. Das Potenzverhalten der Fluktuationsfunktion dient nun zur Bewertung der Langzeitkorrelationen in der Zeitreihe. Man spricht bei $\alpha$ = ½ von unkorrelierten Fluktuationen, z. B. weißes Rauschen, bei $\alpha$ > ½ von positiven Korrelationen und bei $\alpha$ < ½ von negativen Korrelationen. Für den speziellen Fall stationärer, nicht trendbehafteter aber korrelierter Zeitreihen hängt der Fluktuationsexponent über das sog. Wiener-Khintchin-Theorem wieder direkt mit dem Exponenten aus der Autokorrelationsfunktion bzw. dem Exponent aus dem Leistungsspektrum bzw. Potenzspektrum der Fourier-Transformierten der Zeitreihe zusammen.

Bei Zeitreihen aus Immissionsdaten zeigen sich Exponenten von $\alpha$ > ½, d. h. die Fluktuationen in den Emissionen sind nicht zufällig, sondern langzeitkorreliert. Diese Langzeitkorrelation bedeutet schließlich, dass Emissionen eine Persistenz aufweisen, d. h. Emissionen bauen sich nicht instantan ab, sondern verharren eine gewisse Zeit, was als Verharrungsverhalten bzw. Memory-Effekt bezeichnet wird.

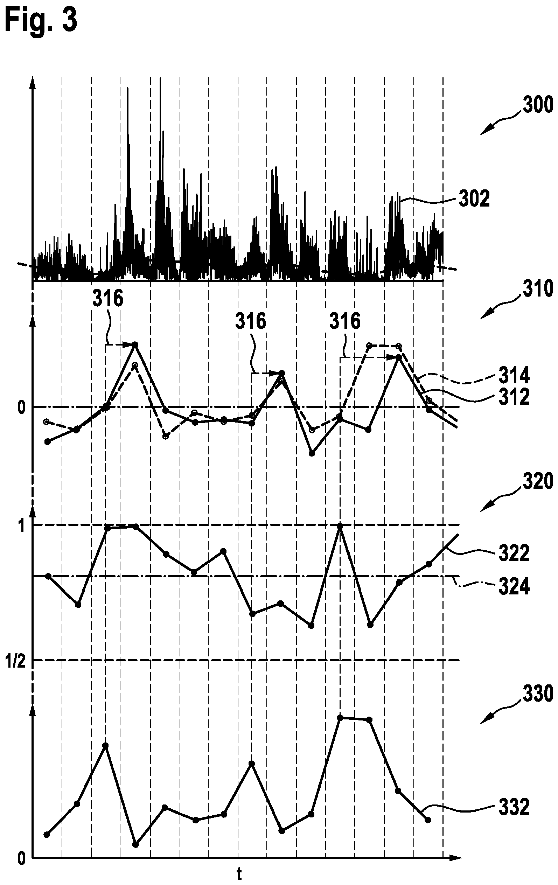

Figur 3 zeigt eine exemplarische Zeitreihe einer Länge von 14 Tagen für Immissionsdaten. In einem ersten Graphen 300 sind Rohdaten 302 $\epsilon$ und Tagesmittelwerte 304 <$\epsilon$> dargestellt. In einem zweiten Graphen 310 sind relative Änderungen $\Delta$<$\epsilon$> der Tagesmittelwerte gegenüber dem Vortag als durchgezogene Linie 312 und eine im Folgenden erläuterte Prognose als gestrichelte Linie 314 gezeigt. Ein dritter Graph 320 zeigt den Verlauf des tagesspezifischen Fluktuationsexponenten $\alpha$ 322. Eine horizontale gestrichelte Linie 324 verdeutlicht den Exponenten für die gesamte Zeitreihe. In einem vierten Graphen 330 ist die betragsmäßige Änderung $|\Delta\alpha|$ 332 des Fluktuationsexponenten gegenüber dem Vortag dargestellt. Starke Änderungen des Exponenten sind hierbei Indikatoren für zukünftige Änderungen in den Immissionen und erscheinen daher zeitlich voranschreitend, wie mit Pfeilen 316 in dem zweiten Graphen 310 verdeutlicht ist.

Betrachtet man nun eine Zeitreihe gemessener Immissionen, z. B. $\epsilon$ für Stickoxid NO im ersten Graphen 300, über einen Zeitraum von mehreren Tagen, so fällt zunächst auf, dass der Verlauf einen ausgeprägten Tag/Nacht-Zyklus hat. Die Immissionen sind tagsüber höher als nachts, was hinsichtlich des jeweiligen Verkehrsaufkommens trivial erscheint. Innerhalb eines Tages können darüber hinaus insbesondere vormittags und nachmittags periodische Überhöhungen beobachtet werden, die auf ein erhöhtes Verkehrsaufkommen zu Stoßzeiten schließen lassen. Folglich ist die Zeitreihe trendbehaftet, so dass für eine Fluktuationsanalyse ein trendbereinigendes Verfahren, wie bspw. die DFA, zweckmäßig ist.

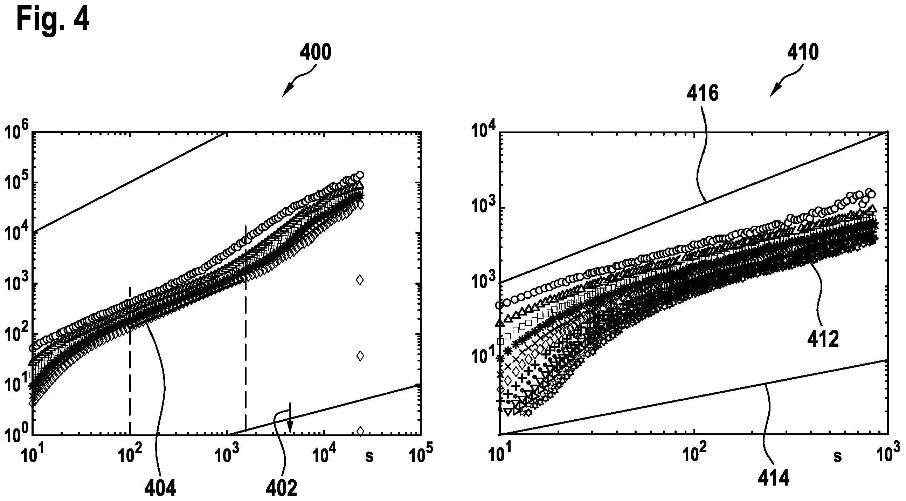

Figur 4 zeigt in einem ersten Graphen 400 Verläufe einer Fluktuationsfunktion für DFA der Ordnungen n = 1, ..., 5 für eine Immissionszeitreihe aus 28 Tagen. Periodizitäten in den Trends, d. h. Oszillationen, führen in der Fluktuationsfunktion zu einem charakteristischen Buckel. Anhand des Buckels, der durch die Trendbereinigung mit Polynomen höherer Ordnung zunehmend verschoben und kleiner wird, kann auf die Periodizität des Trends, in diesem Fall Stoßzeiten vormittags und nachmittags sowie Tag/Nacht, geschlossen und eine Peridendauer T 402 bestimmt werden. Unterhalb und oberhalb des Buckels lässt sich der Fluktuationsexponent bestimmen. Weiterhin zeigt eine Steigung 404 der Fluktuationsfunktion $\alpha = 0{,}82$ für einen längeren Zeitraum, d. h. den Langzeitexponenten, an.

In einem zweiten Graphen 410 ist die Fluktuationsfunktion für DFA der Ordnungen n = 1, ..., 12 für einen spezifischen Tag dargestellt. Der Exponent an einzelnen Tagen wird regelmäßig vom Langzeitexponenten einer mehrtägigen Reihe abweichen. Es wird hierzu auch auf den dritten Graphen 320 in Figur 3 verwiesen. Weiterhin ist im zweiten Graphen 410 der Figur 4 eine erste Steigung 412 für $\alpha = 0{,}717$ eingetragen. Außerdem sind eine zweite Steigung 414 für $\alpha = \frac{1}{2}$ und eine dritte Steigung 416 für $\alpha = 1$ eingetragen. Die Steigungen $\alpha = \frac{1}{2}$ und $\alpha = 1$ dienen lediglich dem Vergleich.

Die in der Zeitreihe ggf. enthaltenen Oszillationen können bei langen Zeitreihen, bspw. über mehrere Tage, zu charakteristischen Abweichungen der Fluktuationsfunktion führen, in diesem Fall einem Buckel, im sonst geraden asymptotischen Verlauf der Fluktuationsfunktion, wie dies in Figur 4 zu erkennen ist. Bei hinreichend vielen Daten kann aufgrund der Lage des Buckels die Periodendauer und die Amplitude abgeschätzt werden. Unterhalb und oberhalb des Buckels zeigt sich der mit der Steigung verknüpfte Fluktuationsexponent.

Mit einer DFA kann bspw. nun eine gewonnene Zeitreihe auf verschiedenen Zeitskalen untersucht werden. So kann z. B. die Langzeitkorrelation für einen Zeitraum über mehrere Tage ermittelt werden. Ebenso kann die Langzeitkorrelation der Fluktuation auch für die Dauer eines Tages, siehe Figuren 3 und 4, ermittelt werden. Da hier regelmäßig nur zwei Überhöhungen am Tag, typischerweise bei Stoßzeiten, vorkommen, ist der periodische Trend bezogen auf die Länge der Zeitreihe "langsam" so dass kein Buckel in der Fluktuationsfunktion erscheint, da die Periodendauer sich außerhalb des mit dieser Länge darstellbaren Skalenbereichs befindet sowie von den verwendeten Polynomen vollständig trendbereinigt wird. Von Tag zu Tag wird der Fluktuationsexponent typischerweise leicht schwanken, siehe hierzu Figur 3, dritter Graph 320. Über mehrere Tage hinweg werden sich die schwankenden Werte regelmäßig um einen Wert bewegen, der dem Fluktuationsexponent $\alpha_{lt}$ (lt für long time) für eine Zeitreihe von mehreren Tagen entspricht, siehe Figur 3, dritter Graph 320.

Betrachtet man dann die absolute Änderung des Fluktuationsexponenten von einem Tag gegenüber dem Vortag, ergeben sich die Erhöhungen und Erniedrigungen $|\Delta\alpha|$ der Fluktuationsexponenten, siehe Figur 3, vierter Graph 330. Betrachtet man dahingegen die Veränderung eines Tagesmittelwerts aus Emissionswerten $<\varepsilon>$ gegenüber dem des jeweiligen Vortags $\Delta\varepsilon$, siehe Figur 3, vierter Graph 330, so fällt auf, dass die Veränderungen des Fluktuationsexponenten $|\Delta\alpha|$ den Änderungen der Tagesmittelwerte der Emissionen $\Delta<NO>$ vorauseilen.

Folglich kann über die Änderung des Fluktuationsexponenten in der Vergangenheit auf die Immissionsänderung in der Zukunft geschlossen werden. Dies bedeutet, dass kurzfristige Änderungen in den Exponenten Warnsignale für eine Änderung in den zukünftigen Immissionen sind. Hierdurch ergibt sich die Chance, durch eine geeignete Abbildung, wie z. B. eine Korrelation aus bereits ermittelten Werten, den weiteren An- oder Abstieg von Immissionen auf Basis der Änderung der Fluktuationsexponenten z. B. am Vortag zu prognostizieren, siehe Figur 3, zweiter Graph 310, gestrichelte Kurve 314.

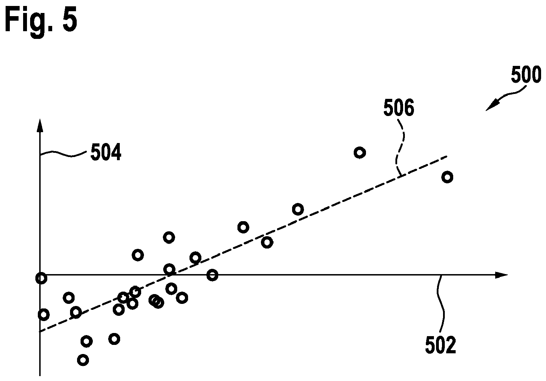

Es wird hierzu auch auf Figur 5 verwiesen, die in einem Graphen 500 eine Korrelation zwischen der Änderung der Emissions-Tagesmittelwerte und der Änderung der Fluktuationsexponenten am jeweiligen Vortag darstellt. Der Graph 500, an dessen Abszisse 502 $|\Delta\alpha|_{t-1}$ aufgetragen ist und an dessen Ordinate 504 zeigt einen approximierten Verlauf 506, der durch

$$\mathrm{Est}\left[\Delta<\varepsilon>_t\right] = a|\Delta\alpha|_{t-1} + b$$

beschrieben ist.

Der Index t steht hier für einen bestimmten Tag. Mit Hilfe einer solchen Regression kann auf Basis des tagesaktuellen Tagesmittelwerts auf den Tagesmittelwert am Folgetag geschlossen werden.

Die DFA kann auch hinsichtlich der Korrelation zwischen mehreren Zeitreihen, wie bspw. NOx, Partikel, Feuchte, Temperatur, Wind, erweitert werden. Dies wird als eine Kreuz-Korrelations-DFA (Cross-correlation DFA) bezeichnet. Hierdurch kann auch ermittelt werden, wie sich z. B. wetterbedingte Einflüsse auf die Korrelation auswirken. Dies kann bspw. dafür verwendet werden, um zum einen die Korrelationsfunktion für die Prognose zu verbessern und zum anderen auch direkt die Änderung auf das Korrelationsverhalten zu erfassen, um die Prognose weiter zu verbessern.

Über eine Kreuzkorrelations-Fluktuationsanalyse zwischen z. B. Zeitreihen aus Immissionen und Zeitreihen aus Ampelphasen oder Zeitreihen aus Verkehrsgeschwindigkeiten können sich zudem Aufschlüsse über den Zusammenhang zwischen Immissionsverhalten und Verkehrsfluss ergeben, welche z. B. eingesetzt werden können, um verkehrsregelnde Maßnahmen hinsichtlich der Reduktion von Emissionen zu optimieren. Ebenso bietet sich als Erweiterung zur DFA noch die sogenannte multifraktale DFA (MF-DFA) an, in der die DFA für fraktale statistische Momente q in der Fluktuationsanalyse, wobei gilt q = 2 in vorstehend genannter Standard-DFA, durchgeführt wird, wodurch insbesondere regionale Unterschiede in den Langzeitkorrelationen durch das sog. multifraktale Spektrum, insbesondere den Zusammenhang $\alpha$ als Funktion von q, noch besser quantifiziert werden können.

Es wird hierin eine Messeinrichtung oder ein Netz aus mehreren, lokal verteilten Messeinrichtungen, die eine Messanordnung bilden, beschrieben. Diese Messeinrichtung ist dazu eingerichtet, Immissionen, wie bspw. Stickstoffmonoxid, Stickstoffdioxid, Kohlenmonoxid, Schwefeldioxid, Ozon und Partikel, sowie weitere relevante Größen, wie z. B. Druck, Temperatur und Feuchte, aber ggf. auch noch weitere Größen, wie z. B. Dauer von Ampelphasen bzw. Umschaltzeitpunkte sowie Geschwindigkeit des Verkehrsflusses oder Anzahl der Fahrzeuge, kontinuierlich, zeitsynchron und zeitlich hoch abgetastet als Zeitreihe, zu erfassen.

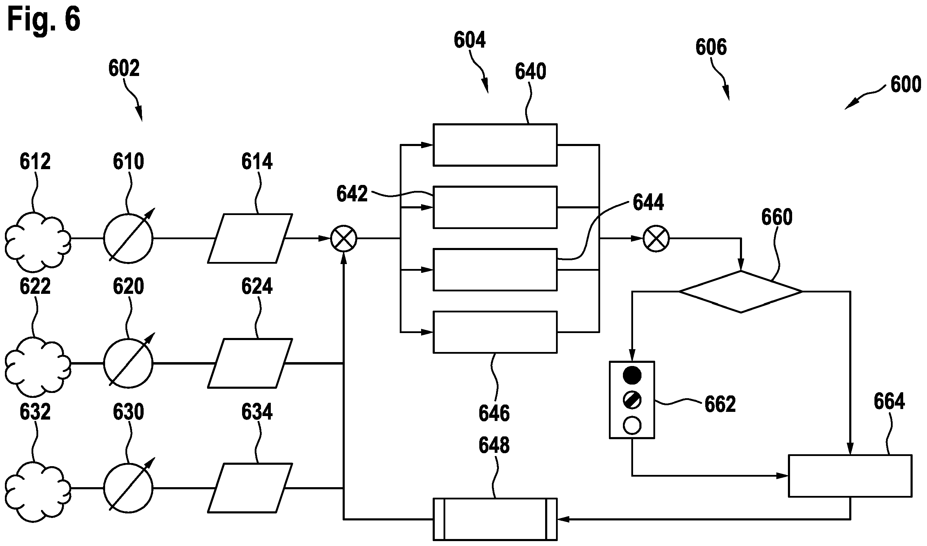

Figur 6 zeigt eine Prinzipskizze einer Regelschleife, die insgesamt mit der Bezugsziffer 600 bezeichnet ist. Die Darstellung zeigt drei Bereiche, nämlich eine Mess- und Kommunikationseinrichtung 602, einen Daten-Analyse-Dienst 604 und ein Verkehrsleitsystem 606. Bei der Mess- und Kommunikationseinrichtung 602 sind ein erstes Messinstrument 610 zum Messen einer Emission 612, das mit einer ersten Datenverarbeitung 614 verbunden ist, ein zweites Messinstrument 620 zum Messen einer Temperatur 622, das mit einer zweiten Datenverarbeitung 624 verbunden ist, und ein drittes Messinstrument 630 zum Messen einer Luftfeuchtigkeit 632, das mit einer dritten Datenverarbeitung 634 verbunden ist, vorgesehen, wobei noch weitere Messinstrumente je nach Zweckmäßigkeit vorhanden sein können.

Der Daten-Analyse-Dienst 604 umfasst Einrichtungen zur Datenanalyse mit Zeitreihenanalyse 640, räumlicher Korrelation 642, Verkehrskorrelation 644, weitere Einheiten 646 und einer Einheit 648 zum Prüfen der Wirksamkeit einer getroffenen Gegenmaßnahme. So verringern sich bspw. eine Änderung der Korrelationen und somit große Änderungen in den Rohimmissionen bei z. B. einem Anpassen der Ampelphasen oder einer Vorgabe einer Geschwindigkeitsbegrenzung.

Das Verkehrsleitsystem 606 umfasst eine Bewertungseinheit 660, die prüft, ob die Änderung der Fluktuationen einen kritischen Schwellenwert übersteigt, der einen Ausstieg der Immissionen am nächsten Tag erwarten lässt (siehe hierzu Figur 3), und die, falls die Bewertung ja ergibt, eine Maßnahme 662, bspw. eine Ampelphasenänderung oder Geschwindigkeitsbegrenzung, vornimmt und andernfalls nur eine weitere Überwachung 664 vornimmt.

Die Daten werden somit in einer Zeitreihe erfasst und bspw. mittels Fluktuationsanalyse ausgewertet. Deutet die Änderung des Fluktuationsexponenten auf eine Erhöhung der am nächsten Tag zu erwartenden Immission hin, so können Maßnahmen, bspw. die Anpassung von Ampelschaltungen oder Geschwindigkeitsbegrenzungen bis hin zu lokalen Durchfahrtsbeschränkungen, tagesaktuell durchgeführt werden. Deuten die Exponenten hingegen auf Entspannung hin, so können Maßnahmen gelockert werden. Die Veränderungen werden erfasst und es werden bspw. mittels Kreuzkorrelations-DFA und/oder mittels Methoden des maschinellen Lernens, z. B. einer Mustererkennung, einer Hauptkomponenten-Analyse o. ä., wirksame Parameter identifiziert und eingelernt.

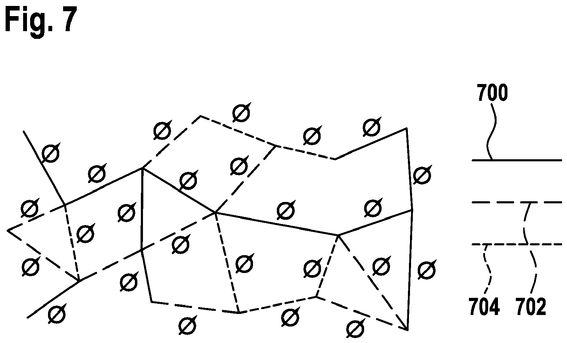

Figur 7 zeigt eine Prinzipskizze lokal ermittelter und geregelter Regionen, in der räumlich verteilte Messsationen bzw. Messknoten an z. B. Straßen dargestellt sind. Die dargestellten Linien repräsentieren bspw. Straßenverläufe. Eine durchgezogene Linie 700 zeigt $\alpha < \alpha_c$, eine erste gestrichelte Linie 702 zeigt $\alpha$ ungefähr gleich $\alpha_c$ und eine zweite gestrichelte Linie 704 zeigt $\alpha > \alpha_c$. D. h. in den Regionen mit $\alpha > \alpha_c$ ist mit einer Erhöhung der Immissionen zu rechnen.

Zu beachten ist, dass die ermittelte Zeitreihe über einen längeren Zeitraum sowie über kürzere Zeiträume, wie bspw. 24 h, erfasst und lokal zwischengespeichert oder als Daten an einen Cloud-Server übermittelt und mittels mathematischer Verfahren zur Fluktuationsanalyse, wie z. B. die DFA, lokal oder in einem Cloud-Server analysiert und das Skalenverhalten der Fluktuation der Messreihe in diesem Zeitraum ausgewertet wird. Die Daten sowie die ermittelten Prognosen werden z. B. in einem Cloud-Server gesammelt und bspw. für die Empfehlung der Einleitung von Maßnahmen an ein Verkehrsleitsystem übergeben. Eine Verkehrsregelung auf Basis der erfassten Daten geschieht dann z. B. wie folgt: Ergeben sich zu einem vorangegangenen Zeitraum, z. B. dem Vortag, starke Änderungen $|\Delta\alpha|$ im Exponenten der für die Zeitreihe an diesem Tag ermittelten Fluktuationsfunktion $F^{(n)}(s) \propto s^{\alpha}$, so ist dies ein Indikator für eine zu erwartende Änderung der Immissionen, z. B. $\Delta$<NO>, in unmittelbarer Zukunft, siehe Figur 3, zweiter Graph 310. Je nach Abweichung kann dann, wie dies in Figur 6 dargestellt ist, vorgegangen werden. Sind die Änderungen klein, so wird regelmäßig die Immission abnehmen und es sind keine weiteren als die bisherigen Maßnahmen erforderlich. Je nach vorangegangenem Immissionsniveau können dann ggf. auch Maßnahmen aufgehoben werden. Die Bewertung erfolgt ggf. durch die Bewertung weiterer Prognosen, wie z. B. die Änderung der Verkehrsdichte und/oder die Änderung in den Wetterverhältnissen. Sind die Änderungen im Fluktuationsexponenten hingegen hoch, so lässt dies auf eine zu erwartende Erhöhung der Immissionen schließen. In diesem Fall werden, ggf. auch räumlich beschränkt, Maßnahmen ergriffen, z. B. Geschwindigkeitsbegrenzung, Anpassung von Ampelanlagen, Durchfahrtssperrung einzelner Straßen. Die ergriffene Maßnahme kann abhängig von der Stärke der Fluktuation sowie weiterer Merkmale angepasst sein. Über die weitere Überwachung der Immissionen kann über die Fluktuationsanalyse und die mittleren Tagesverläufe, d. h. Tagesmittelwert, der Erfolg der Maßnahme bewertet werden. Es wird hierzu auf Figur 6 verwiesen.

Abhängig von lokalen Gegebenheiten, z. B. der Art der Bebauung, räumliche Nähe weiterer Emissionsquellen, wie bspw. Industrieanlagen usw., können sich die Fluktuationen und somit die zu erwartenden Immissionen bzw. deren zeitliches Verhalten stark unterscheiden. Zudem ist anzumerken, dass sich Emissionen, z. B. NO, von denen bspw. aus Partikelemissionen hinsichtlich ihrer Persistenz stark unterscheiden können und dies insbes. auch räumlich unterschiedlich sein kann. So können Partikelemissionen z. B. durch Niederschlag und/oder Wind sowie durch Agglomeration und Niedersinken schneller ausgetragen werden. Um solche lokalen Unterschiede zu bewerten, können die Messungen sowie die Analyse der Daten und die daraus hervorgehende Prognose auch über ein Netzwerk aus verschiedenen räumlich verteilten Messknoten erfolgen, siehe hierzu Figur 7.

Dabei können die Messknoten aus z. B. miniaturisierten Messboxen bestehen. Diese umfassen neben geeigneten, insbesondere hinsichtlich Auflösung, Genauigkeit und Stabilität, Sensoren für die gewünschten zu beobachtenden Größen, wie z. B. NOx, CO2, Partikel PM10 und/oder PM2,5 sowie Temperatur, relative Feuchte, Niederschlag, Wind usw., einen Datenspeicher für die temporäre Speicherung der Zeitreihe und/oder eine geeignete Vorrichtung, wie z. B. ein Mobilfunkknoten zum Transfer der Sensordaten bspw. auf einen Großrechner. Die Daten können mittels einer Rechnereinheit entweder lokal analysiert oder im Falle transferierter Rohdaten extern in einem Großrechner analysiert werden.

Das Ermitteln räumlich ggf. unterschiedlicher Prognosen erlaubt es, erforderliche Maßnahmen frühzeitig lokal und tagesaktuell anzupassen. Aus der langfristigen Analyse der Persistenz von Immissionen können sich zudem lokale Optimierungsmöglichkeiten ableiten lassen. So kann z. B. bei lokal hochkorrelierten Immissionszeitreihen eine bauliche Änderung, z. B. eine Änderung der Bebauung und/oder eine Straßenverlegung, empfehlenswert sein. Darüber ist es möglich, die Änderung von Antriebskonzepten, z. B. die zunehmende Zahl von elektrobetriebenen Fahrzeugen, hinsichtlich des Persistenzverhaltens der Emissionen zu untersuchen bzw. durch das Korrelationsverhalten auch anderen Emissionsquellen hinsichtlich ihren Emissionsdynamik, wie bspw. jahreszeitlich bedingte Änderung zeitlicher Korrelationen durch Erhöhung der Emissionen aus Haushalten, z. B. Heizungsanlagen, zu beobachten und zu bewerten, um ein besseres Verständnis über das Entstehen und das räumliche sowie zeitliche Verhalten von Emissionen zu erlangen, um somit geeignete Maßnahmen und Strategien zur Senkung der Emissionen insbesondere in Ballungsgebieten zu ermöglichen.

Das vorgestellte Verfahren kann insbesondere im Rahmen eines Dienstes, bspw. für Städte, eingesetzt werden. Der Einsatz von Messstationen bzw. Messboxen soll dabei ermöglichen, zeitlich und räumlich hoch aufgelöste Daten zu Immissionen zur Verfügung zu stellen und daraus Empfehlungen, bspw. für die Verkehrssteuerung, abzuleiten und die Wirkungen der Maßnahmen zu überwachen.

## Patentansprüche

1. Verfahren zum Auswerten mindestens eines Signals (202), das eine Zeitreihe von Messwerten repräsentiert, wobei die Messwerte Immissionen betreffen, wobei das mindestens eine Signal (202) mittels einer trendbereinigenden Fluktuationsanalyse ausgewertet wird, so dass ein trendbereinigtes Signal erhalten wird, das wiederum ausgewertet wird, um eine zeitliche Veränderung der Fluktuation der Messwerte zu erkennen und diese zeitliche Veränderung auszuwerten.

2. Verfahren nach Anspruch 1, bei dem das mindestens eine Signal (202) mit einer DFA ausgewertet wird.

3. Verfahren nach Anspruch 1 oder 2, bei dem eine erkannte zeitliche Veränderung in der Fluktuation dazu genutzt wird, um auf zukünftige Ereignisse, die die Immissionen betreffen, zu schließen.

4. Verfahren nach Anspruch 3, bei dem in Abhängigkeit von dem zukünftigen Ereignis Maßnahmen eingeleitet werden.

5. Verfahren nach Anspruch 4, bei dem das trendbereinigte Signal dazu verwendet wird, um mit Methoden des maschinellen Lernens Wirkungen der eingeleiteten Maßnahmen zu bewerten.

6. Verfahren nach einem der Ansprüche 1 bis 5, bei dem eine Kreuzkorrelation durchgeführt wird.

7. Verfahren nach einem der Ansprüche 1 bis 6, bei dem das mindestens eine Signal (202) kumuliert wird, so dass sich eine kumulierte Reihe (212) ergibt, die kumulierte Reihe (212) sukzessive in Fenster der Breite s (228) segmentiert wird und in jedem Fenster die kumulierte Reihe (212) durch ein Polynom (222) n-ten Grades angepasst wird, dann ein Residuum (224) zwischen der kumulierten Reihe (212) und dem Polynom (222) ermittelt wird und die Fluktuation des Residuums (224) in jedem Fenster der Breite s (228) berechnet und über die Anzahl der Fenster gemittelt wird, wobei dies für verschiedene Fensterbreiten wiederholt wird, so dass eine Fluktuationsfunktion $F^{(n)}(s)$ ermittelt wird, die im Langzeitverhalten ein Potenzgesetz $F^{(n)}(s) \propto s^{\alpha}$ wiedergibt und ein

Fluktuationsexponent $\alpha$ (322) ausgewertet wird.

8.  Verfahren nach Anspruch 7, bei dem Veränderungen des Fluktuationsexponenten gegenüber dem Fluktuationsexponenten (322) vom Vortag ausgewertet werden, um auf eine Änderung der Immissionen in der Zukunft zu schließen.

9.  Anordnung zum Auswerten mindestens eines Signals, die zur Durchführung eines Verfahrens nach einem der Ansprüche 1 bis 8 eingerichtet ist.

10. Anordnung nach Anspruch 9, die in einer Messstation (12, 14, 16, 18) integriert ist.

**Fig. 1**

EP 3 712 786 A1

Fig. 2

# Fig. 3

Fig. 4

## Fig. 5

Fig. 6

# Fig. 7

Europäisches
Patentamt

European
Patent Office

Office européen
des brevets

# EUROPÄISCHER RECHERCHENBERICHT

**Nummer der Anmeldung**

EP 20 16 4529

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CHAKRABORTHY PARTHASARATHI ET AL: "Investigation of power-law correlations within daily total ozone time series and search for a stable node through phase portrait", COMPTES RENDUS GEOSCIENCE, ELSEVIER, PARIS, FR, Bd. 345, Nr. 2, 22. Januar 2013 (2013-01-22), Seiten 55-61, XP028580641, ISSN: 1631-0713, DOI: 10.1016/J.CRTE.2012.12.002 * Sektionen 1, 2.1, 2.2; Tabelle 1 * ----- | 1-10 | INV. G06F17/18 |
| X | Jaross Law Kwapie ET AL: "Detrended fluctuation analysis made flexible to detect range of cross-correlated fluctuations", , 10. November 2015 (2015-11-10), Seite 70, XP055723351, Gefunden im Internet: URL:https://arxiv.org/pdf/1506.08692.pdf [gefunden am 2020-08-18] * Zusammenfassung * ----- | 1-10 | **RECHERCHIERTE SACHGEBIETE (IPC)**<br><br>G06F |

Der vorliegende Recherchenbericht wurde für alle Patentansprüche erstellt

| Recherchenort | Abschlußdatum der Recherche | Prüfer |
|---|---|---|
| Berlin | 18. August 2020 | Virnik, Elena |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)